# EUROPEAN PATENT SPECIFICATION

(11) **EP 3 342 902 B1**
(45) Date of publication and mention of the grant of the patent: **19.05.2021**
(21) Application number: 17155465.2
(22) Date of filing: 09.02.2017
(51) Int. Cl.: D01F 1/10, C08J 3/22

(54) **SYNTHETIC FIBER OR FILAMENT WITH HERBAL RESIDUE AND METHOD OF MAKING THE SAME**
SYNTHETISCHE FASER ODER DAS FILAMENT MIT KRÄUTERS RÜCKSTANDS UND VERFAHREN ZU IHRER HERSTELLUNG
FIBRE SYNTHÉTIQUE OU FILAMENT À RÉSIDUS D'HERBES ET SON PROCÉDÉ DE FABRICATION

(30) Priority: 27.12.2016 IN 201621044524
(43) Date of publication of application: 04.07.2018
(73) Proprietor: AYM Syntex Ltd, 400013 Mumbai (IN); Mandawewala, Abhishek, 400013 Mumbai (IN)
(72) Inventor: MANDAWEWALA, ABHISHEK, 400013 Mumbai (IN); KUMAR PATHAK, PRAMOD, 396230 UT of Dadra & Nagar Haveli (IN); VERMA, PANKAJ, 400013 Mumbai (IN)
(74) Representative: Sach, Greg Robert

(56) References cited:
- CA-A1- 2 647 567
- CN-A- 104 088 030
- US-A1- 2013 034 620
- US-A1- 2013 203 919

## Description

### TECHNICAL FIELD

Embodiments described herein relate to a non-natural fiber or filament composition comprising a herbal residue. The embodiments further relate to an herbal masterbatch made with the use of natural and/or herbal products, polymers and additives. More particularly the embodiments described herein relate to the masterbatch for use in manufacturing of chemical-free non-natural fiber or filament having additional beneficial properties such as anti-microbial & anti-odor behavior.

### BACKGROUND

Textile industry has conventionally been using both natural and synthetic fibers for manufacture of various products. Each having their own advantages and shortcomings related to strength, comfort, softness, longevity and the like. With ever-increasing demand to provide enhanced quality fibers manufacturers apart from enhancing traditional properties, have started incorporating more functional elements to fibers to provide better comfort, freshness, and hygiene to users. In pursuit of meeting demands of modern hygienic lifestyle, there have been several attempts to add a variety of chemical agents to fabrics providing them with anti-microbial properties. These agents are normally applied on the surface to impart anti-microbial capabilities to the fabrics during finishing stage. However, items made with such fabrics are unable to retain anti-microbial capabilities for long and as the compounds are washed-off during washings or ultra-violet exposure. A significant reason that these fibers are not able to provide long-term anti-microbial effects lies on the fact that these components are added to the fiber during the fabric finishing stage through the use of various finishing chemicals. Moreover, there is always a concern regarding use of such chemical agents and their potential after-effects on human body.

One way of overcoming this problem is through use of a masterbatch during the initial stages of fiber production, thereby adding longevity to the functional benefit to the fabrics made from such fibers. A masterbatch (MB) is an additive used for obtaining multiple functional benefits to the synthetic fibers. However, chemical masterbatches are expensive, and a different additive is required to obtain each different function. Therefore, there exists a need for innovation in relation to this technology to overcome the shortcomings prevalent in the market.

The present invention provides a non-natural fiber or filament offering functional benefits such as anti-microbial, anti-odor, cooling effect, and the like and method of preparing the same. The present invention also provides a natural and herbal masterbatch to be used in the preparation of the non-natural fiber or filaments and method of preparing the same. An objective of the present invention is to use inexpensive natural and herbal products such as neem, babool, etc. in production of non-natural fiber or filaments to provide beneficial properties such as anti-microbial, anti-odor, cooling effect and the like.

CN107088030A discloses an outdoor mosquito repellent artificial yarn fiber. The outdoor mosquito repellent artificial yarn fiber is prepared from the following components in parts by weight: 70-90 parts of macromolecule olefin resin, 5-20 parts of mosquito repellent Chinese herbal medicine master batch, 0-5 parts of a toughening agent, 0-5 parts of an anti-aging agent, 0-5 parts of an antistatic agent and 2-6 parts of color master batch, wherein the mosquito repellent Chinese herbal medicine master batch is prepared from the following components in parts by weight: 60-80 parts of macromolecule matrix resin, 10-40 parts of mosquito repellent Chinese herbal medicine powder and 0-5 parts of a processing agent. The Chinese herbal medicine powder material with mosquito repellent effect is added in the formula, the mosquito repellent Chinese herbal medicine in the artificial yarn can release substance with mosquito repellent effect under the natural condition to form a protective layer above the artificial lawn; the outdoor mosquito repellent artificial yarn fiber can effectively repel the mosquito and is harmless to human body, and the fragrant Chinese herbal medicine flavor makes people relax and happy, and the mosquito repellent at summer night is especially obvious.

US2013203919A1 discloses a synthetic fiber comprising a plant fatty acid and a method for manufacturing the same. The method comprises incorporating a plant fatty acid in an amount of from 0.1 to 10.0 wt % into a fiber-formable polymer; and melt-spinning the plant fatty acid-incorporated polymer. The synthetic fiber comprises a plant fatty acid in an amount of from 0.01 to 1.0.0 wt %, and emanates a plant fragrance. In addition to being superior to general synthetic fibers in physical properties including strength and elongation, the synthetic fiber exhibits excellent bulkiness, elasticity, whiteness, touch sensation, hygroscopicity, dyeability, and gloss. Further, the fiber is highly antistatic and gives off a plant fragrance, so that it is useful as a material for high-quality clothes.

US2013034620A1 discloses an antibacterial synthetic fiber, and a method for manufacturing the same, characterized in that one or more antibacterial plant extracts are mixed with a fiber-formable polymer and the mixture is melt spun at 200^{∼}300° C. The antibacterial synthetic fiber exhibits excellent and persistent antibacterial activity. In addition, the antibacterial synthetic fiber is superior in physical property to conventional antibacterial fibers and is suitable for use as a material for clothes.

CA2647867 discloses a process for producing porous polymer masterbatch having anti-bacteria and odor eliminating functions, wherein said fiber contains materials such as porous natural mineral kieselguhr or active carbon, can adsorb and eliminate odor such as stink of sweat and the like discharged from human body, has functions of sterilization, anti-bacteria, anti-mould, odor eliminating and the like. Pores of the natural mineral kieselguhr or active carbon contain organic Chinese herbal medicine and inorganic anti-bacteria minerals, wherein all of such organic Chinese herbal medicine and inorganic anti-bacteria minerals have functions of anti-bacteria, anti-fungus and the like, can eliminate effectively odor or reduce substantially stink, and can be applied extensively on various fabrics, clothes and ornaments or other goods.

### SUMMARY

This Summary is provided to introduce a selection of concepts in a simplified form that are further described below in the Detailed Description. This Summary is not intended to identify key features or essential features of the claimed subject matter, nor it is intended to be used to limit the scope of the claimed subject matter.

An object of the present invention is to provide a non-natural fiber or filament composition and/or a non-natural fiber or filament, exhibiting functional benefits such as anti-microbial, anti-odor, cooling effect, and the like.

An object of the present invention is to provide a method of manufacturing a non-natural fiber or filament composition and/or a non-natural fiber or filament, exhibiting functional benefits such as anti-microbial, anti-odor, cooling effect, and the like.

An object of the present invention is to use inexpensive natural and herbal products or processed herbal products in the manufacture of non-natural fiber or filaments to exhibit functional benefits such as anti-microbial, anti-odor, cooling effect and the like.

An object of the present invention is to manufacture a non-natural fiber or filament exhibiting functional benefits comprising a natural and herbal masterbatch composition.

Another object of the present invention is to manufacture a natural and herbal masterbatch composition, comprising natural and herbal products, polymers and additives such as coupling agent, UV stabilizer, antioxidant, antistatic agent or colorants in high speed mixer or mixers thereof.

Another object of the present invention is to manufacture non-natural fiber or filaments without the use of chemical agents for providing functionally beneficial properties such as anti-microbial, anti-odor, cooling effect and the like.

A yet another object of the present invention is to introduce a plurality of functional benefits to a non-natural fiber for users.

The present invention further provides a fabric comprising the non-natural fiber or filament composition and/or a non-natural fiber or filament of the present invention having the non-natural fiber or filament having linear density range between 0.33 dtex to 33.3 dtex (denier range between 0.3 DPF to 3 DPF).

### DETAILED DESCRIPTION

The presently disclosed subject matter is described with specificity to meet statutory requiremnts. However, the description itself is not intended to limit the scope of this patent. Rather, the inventors have contemplated that the claimed subject matter might also be embodied in other ways, to include different steps or elements similar to the ones described in this document, in conjunction with other present or future technologies.

It is to be understood that both the foregoing general description and the following detailed description are exemplary and explanatory only, and are not restrictive of the invention, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements and components comprising one unit and elements or components that comprise more than one unit unless specifically stated otherwise.

Reference in this specification to "one embodiment" or "an embodiment" means that a particular feature, structure, or characteristic described in connection with the embodiment is included in at least one embodiment of the disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments.

According to an aspect of the present disclosure, there is provided a non-natural fiber or filament composition. The non-natural fiber or filament composition comprises an herbal masterbatch. The herbal masterbatch further comprises an herbal extract (an extract of at-least one natural or herbal products) of particle size < 50 microns, a polymer powder or a polymer, and one or more from the additives such as coupling agent, UV stabilizer, antioxidant, Antistatic agent, colorants, pigments, and the like. The herbal masterbatch may further comprises a processed extract or a raw extract or a mixture of processed and raw extract of at-least one natural or herbal products. The herbal masterbatch further provides multiple functional benefits such as anti-microbial, anti-odor, cooling effect, etc. to the non-natural fiber or filament.

Further, the natural or herbal products used in making of herbal masterbatch composition preferably comprises of plant extracts or processed plant extract or a mixture of the same. The plant or processed plant extracts used majorly comprises of plants that have medicinal, savory, aromatic, anti-microbial or anti-odor qualities. The plants comprising of these properties includes plants for example, Azadirachta indica, Curcuma longa, Prickly acacia, Mentha piperita, Margosa, Oolong tea, oregano, magnolia, cranberry, rosemary, Camellia, morin, Garcinia mangostana L., Jabara, Juglans regia, Zanthoxylum alantum, Mimusops elengi, Hibiscus abelmoschus, Ayurvedic, Carapa procera, Khaya senegalensis, Salvadora persica, Cucurbitaceae (Citrullus colocynthis) and mixtures thereof.

Any suitable plant extract can be used so long as it enhances the desired effects and other suitable plants for this purpose include but not limited to Acacia catechu, Acacia nilotica, Achyrathes aspera, Aristolochia bracteolate, Cinnamomum camphora, Cinnamomum verum, Curcuma longa, Eucalyptus globulus, Ficus bengalensis, Juglans regia, Madhuca longifolia, Mimusops elengi, Ocimum sanctum, Oolonga tea, Piper betel leaves, Piper longum, Piper nigrum, Potentilla fulgens, Syzygium aromaticum, Spilanthes calva, Vaccinium macrocarpon, Zanthoxylum armatum, and mixtures thereof.

Additional extracts can be selected from one or more plants of the following genera: Zingiberaceae, Mentha, Origanum Thymus, Lavandula, Salvia, Melissa, Cuminum, Petroselinum, Calendula, Tagetes, Boswellia, Sambucus, Copaifera, Curcuma, Allium, Symphytum, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Piper, Syzygium, Commiphora, Juglans, Scutellaria, and Magnolia.

In one of the embodiments, the herbal masterbatch is in the form of powder. The reduction of herbal masterbatch in the form of powder is done as a process of preprocessing of at least one natural or herbal product. Further, the average size of powder is reduced to less than 50 microns or more preferably less than 25 microns to increase in the properties, such as anti-microbial, anti-order, cooling effects etc. of the non-natural fiber or filament composition.

In one of the embodiments, the non-natural fiber or filament comprises the herbal extract, the coupling agent, the polymer, and one or more from the additives such as coupling agent, UV stabilizer, antioxidant, Antistatic agent, colorants, pigments, and the like, in a pre-defined ratio. The pre-defined ratio is 0.1-5% by weight of the at-least one of natural or herbal products, 1-3% by weight of the coupling agent and 80-98% by weight of the base polymer.

In one of the embodiments, the non-natural fiber or filament (final fiber) having a linear density range between 0.33 dtex to 33.3 dtex (denier range between 0.3 DPF to 3 DPF).

In one of the embodiments, the coupling agent is also added to the composition where the coupling agent aids in adhesion of the herbal extract and the polymer. It is to be appreciated that the coupling agents described herein are exemplary in nature, and any suitable coupling agent known to those of ordinary skill in the art may be utilized in any of the exemplary embodiments described or otherwise suggested herein. In some exemplary embodiments, the coupling agent, or coupling agents, may be Silane, Epoxysilane or Organosilane coupling agents. Besides their role of coupling the surface of the reinforcement fibers and the surrounding matrix, coupling agents also function to reduce the level of fuzz, or broken fiber filaments, during subsequent processing.

In one of the embodiments, the polymer or the base polymer is a thermoplastic polymer resin preferably of the polyester family selected from a group of PET, PBT, PTT, PLA, PP, Polyamide or a mixer thereof.

According to another aspect of the invention, there is provided a method of preparing an herbal masterbatch for producing a non-natural fiber or filament exhibiting functional benefits such as anti-microbial, anti-odor, cooling effect, and the like. The method comprises the steps of preparing a natural or herbal masterbatch and drawing a yarn or filament or fiber from the masterbatch to manufacture non-natural fiber or filament, wherein the amount of herbal extract in the non-natural fiber or filament (final fiber) is in a range 0.1-5% by weight.

Further, the method of preparing the natural or herbal masterbatch, comprises th steps of grinding (or processing) at-least one of a herbal or natural products to produce a herbal extract having an average particle size of less than or equal to 50 microns, or more preferably less than 25 microns, and mixing or blending the herbal extract with a coupling agent, a polymer powder, and one or more from the additives such as Coupling agent, UV stabilizer, antioxidant, Antistatic agent, colorants, pigments, and the like, in a high speed mixer.

The class of materials used as UV stabilizer includes but is not limited to: ultraviolet absorbers, quenchers, and hindered amine light stabilizers. These compounds are used to prevent degradation of the polymer due to solar ultraviolet radiation exposure.

In one of the embodiments, the non-natural fiber or filament composition comprising the herbal masterbatch and the polymer is extruded together through conventional spinning processes known in the art to form the non-natural fiber or filament. In one of the exemplary embodiments, the blended mix may be extruded through twin screw extruder followed by melt blending to produce fibers or filaments having denier range between 0.3 DPF to 30DPF.

According to one another aspect of the invention, there is provided a method of manufacturing a non-natural fiber or filament exhibiting functional benefits, comprising the steps of: preparing a natural or herbal masterbatch, blending or re-mixing the herbal masterbatch, the polymer, and one or more from the additives such as coupling agent, UV stabilizer, antioxidant, Antistatic agent, colorants, pigments, and the like to produce a masterbatch; and extruding the produced masterbatch through conventional spinning processes known in the art to manufacture non-natural fiber, wherein the amount of herbal extract in the extruded non-natural fiber is in a range 0.1-5% by weight.

In one of the exemplary embodiments, the herbal masterbatch may mixed with at-least one other masterbatch and extruded to introduce a plurality of functional benefits to a non-natural fiber for users. The other masterbatch may be primarily a color masterbatch, anti-UV masterbatch, anti-static masterbatch and the like. In one of the embodiments, a single herbal masterbatch may be used to impart multiple functional benefits to the non-natural fiber or filament.

In one of the embodiments, the non-natural fiber or filament made using herbal masterbatch is a filament yarn or staple fiber.

In one of the embodiments, the non-natural fiber or filament made using herbal masterbatch is used to manufacturing of knitted, woven or non-woven fabrics.

In one of the embodiments, the non-natural fiber or filament made using herbal masterbatch is relatively inexpensive than a chemical added non-natural fiber or filament. The difference in cost may be attributed to the use of natural or herbal products instead of chemicals.

While the embodiments have been described in connection with the preferred embodiments of the various figures, it is to be understood that other similar embodiments may be used or modifications and additions may be made to the described embodiment for performing the same function without deviating therefrom. Therefore, the disclosed embodiments should not be limited to any single embodiment, but rather should be construed in breadth and scope in accordance with the appended claims.

## Claims

1. A non-natural fiber or filament composition for manufacturing of knitted, woven or non-woven fabrics, the non-natural fiber or filament composition comprising:
a. an herbal masterbatch, wherein the herbal masterbatch comprises:
i. an herbal extract having average particle size of less than or equal to 50 microns, wherein the herbal extract comprises an extract of at-least one natural or herbal products;
ii. at-least one coupling agent; and
iii. polymer, wherein the ratio of the herbal extract to the polymer is 30:70 by wt. in the prepared natural or herbal masterbatch; and
b. a base polymer;
wherein the herbal masterbatch in a form of a filament yarn or a staple fiber and the base polymer is extruded together to form the non-natural fiber having at-least one of an anti-microbial property, an anti-odor property, a cooling property, and,
wherein the amount by weight of herbal extract in the final non-natural fiber is between 0.1-5%.

2. The non-natural fiber or filament composition as claimed in claim 1, wherein the non-natural fiber or filament having a linear density range between 0.33 dtex to 33.3 dtex (denier range between 0.3 DPF to 3 DPF).

3. The non-natural fiber or filament composition as claimed in claim 1, wherein the herbal extract is in a form of powder.

4. The non-natural fiber or filament composition as claimed in claim 1, wherein the polymer or the base polymer is selected from a group of PET, PBT, PTT, PLA, PP, Polyamide or a mixture thereof.

5. The non-natural fiber or filament composition as claimed in claim 1, wherein the coupling agent couples the natural or herbal extract and the base polymer.

6. A method of manufacturing anti-microbial, anti-odor non-natural fiber or filament comprising the steps of:
a. preparing a natural or herbal masterbatch , wherein the natural or herbal masterbatch preparation comprising the steps of:
i. grinding at-least one herbal or natural products to produce a herbal extract having an average particle size of less than or equal to 50 microns; and
ii. mixing or blending the herbal extract, a coupling agent and a polymer to produce a masterbatch, wherein the ratio of the herbal extract to the polymer is 30:70 by wt. in the prepared natural or herbal masterbatch; and
b. extruding the masterbatch in a form of a yarn or filament or fiber and a base polymer together to obtain the final non-natural fiber or filament, wherein the amount of herbal extract in the final non-natural fiber or filament is between 0.1-5% by weight.

7. A method of manufacturing a natural or herbal masterbatch as claimed in claim 1 comprising the steps of:
a. grinding at-least one herbal or natural products to produce an herbal extract having an average particle size of less than or equal to 50 microns; and
b. mixing or blending the herbal extract, a polymer and coupling agent to manufacture natural or herbal masterbatch, wherein the ratio of the herbal extract to the polymer is 30:70 by wt. in the prepared natural or herbal masterbatch.

8. A method as claimed in claim 7, wherein the coupling agent comprise at-least a coupling agent, UV stabilizer, antioxidant, antistatic agent or colorants in high speed mixer or mixers thereof.

9. The method as claimed in claim 7, wherein the manufactured non-natural fiber comprises at-least one of an anti-microbial property, an anti-odor property, a cooling property, and durability property.

10. The method as claimed in claim 7, wherein the herbal masterbatch is mixed with at-least another masterbatch and extruded to introduce a non-natural fiber.

## Patentansprüche

1. Eine nichtnatürliche Faser- oder Filamentzusammensetzung zur Herstellung von gestrickten, gewebten oder nicht-gewebten Stoffen, wobei die nichtnatürliche Faser- oder Filamentzusammensetzung umfasst:
a. Ein pflanzlicher Masterbatch, wobei der pflanzliche Masterbatch, umfasst:
i. einen Pflanzenextrakt mit einer durchschnittlichen Teilchengröße von weniger als oder gleich 50 Mikrometern, wobei der Pflanzenextrakt einen Extrakt von mindestens einem natürlichen oder pflanzlichen Produkt umfasst;
ii. mindestens ein Haftvermittler; und
ili. ein Polymer, wobei das Verhältnis des Pflanzenextrakts zu dem Polymer 30:70 Gew. in dem hergestellten natürlichen oder pflanzlichen Masterbatch beträgt; und
b. ein Basispolymer;
wobei der pflanzliche Masterbatch in Form eines Filamentgarns oder einer Stapelfaser und das Basispolymer zusammen extrudiert werden, um die nichtnatürliche Faser zu bilden, die mindestens eine Eigenschaft besitzt, die antimikrobielle, geruchshemmenden, kühlend ist, und,
wobei die Gewichtsmenge des Pflanzenextrakts in der fertigen nichtnatürlichen Faser zwischen 0,1-5 % liegt.

2. Die nichtnatürliche Faser- oder Filamentzusammensetzung nach Anspruch 1, wobei die nichtnatürliche Faser oder das nichtnatürliche Filament einen linearen Dichtebereich zwischen 0,33 dtex bis 33,3 dtex (Denier-Bereich zwischen 0,3 DPF bis 3 DPF) aufweist.

3. Die nichtnatürliche Faser- oder Filamentzusammensetzung nach Anspruch 1, wobei der Pflanzenextrakt in Form von Pulver vorliegt.

4. Nichtnatürliche Faser- oder Filamentzusammensetzung nach Anspruch 1, wobei das Polymer oder das Basispolymer aus einer Gruppe von PET, PBT, PTT, PLA, PP, Polyamid oder einer Mischung davon ausgewählt ist.

5. Die nichtnatürliche Faser- oder Filamentzusammensetzung nach Anspruch 1, wobei der Haftvermittler den natürlichen oder Pflanzenextrakt und das Basispolymer verbindet.

6. Ein Verfahren zur Herstellung einer antimikrobiellen, geruchshemmenden nichtnatürlichen Faser oder eines Filaments, diese Schritte umfassend:
a. Herstellen eines natürlichen oder pflanzlichen Masterbatches, wobei die Herstellung des natürlichen oder pflanzlichen Masterbatches die folgenden Schritte umfasst:
i. Mahlen mindestens eines pflanzlichen oder natürlichen Produkts, um einen Pflanzenextrakt mit einer durchschnittlichen Teilchengröße von weniger als oder gleich 50 Mikrometern herzustellen; und
ii. Mischen oder Vermengen des Pflanzenextrakts, eines Haftvermittlers und eines Polymers zur Herstellung eines Masterbatch, wobei das Verhältnis des Pflanzenextrakts zu dem Polymer 30:70 nach Gewicht in dem hergestellten natürlichen oder pflanzlichen Masterbatch beträgt; und
b. Extrudieren des Masterbatch in Form eines Garns oder Filaments oder einer Faser und eines Basispolymers zusammen, um die endgültige nichtnatürliche Faser oder das endgültige nichtnatürliche Filament zu erhalten, wobei die Menge des Pflanzenextrakts in der endgültigen nicht-natürlichen Faser oder dem endgültigen nichtnatürlichen Filament zwischen 0,1-5 Gew.% liegt.

7. Verfahren zur Herstellung eines natürlichen oder pflanzlichen Masterbatches nach Anspruch 1, umfassend die Schritte:
a. Mahlen mindestens eines pflanzlichen oder natürlichen Produkts zur Herstellung eines Pflanzenextrakts mit einer durchschnittlichen Teilchengröße von weniger als oder gleich 50 Mikrometern; und
b. Mischen oder Vermengen des Pflanzenextrakts, eines Polymers und eines Haftvermittlers zur Herstellung von natürlichem oder pflanzlichem Masterbatch, wobei das Verhältnis des Pflanzenextrakts zu dem Polymer 30:70 Gew.% in dem hergestellten natürlichen oder pflanzlichen Masterbatch beträgt.

8. Verfahren nach Anspruch 7, wobei der Haftvermittler mindestens einen Haftvermittler, einen UV-Stabilisator, ein Antioxidationsmittel, ein antistatisches Mittel oder Farbstoffe in einem Hochgeschwindigkeitsmischer oder Mischern davon umfasst.

9. Verfahren nach Anspruch 7, wobei die hergestellte nichtnatürliche Faser mindestens eine der folgenden Eigenschaften aufweist: antimikrobiell, geruchshemmend, kühlend und haltbar.

10. Verfahren nach Anspruch 7, wobei das pflanzliche Masterbatch mit mindestens einem anderen Masterbatch gemischt und extrudiert wird, um eine nichtnatürliche Faser einzuführen.

## Revendications

1. Composition de fibres ou de filaments non naturel(le)s pour la fabrication de tissus tricotés, tissés ou non tissés, la composition de fibres ou de filaments non naturel(le)s comprenant :
a. un mélange-maître à base de plantes, le mélange-maître à base de plantes comprenant :
i. un extrait à base de plantes ayant une taille moyenne des particules inférieure ou égale à 50 microns, l'extrait à base de plantes comprenant un extrait d'au moins un produit naturel ou à base de plantes ;
ii. au moins un agent de couplage ; et
iii. polymère, dans lequel le rapport de l'extrait à base de plantes sur le polymère est de 30:70 en poids dans le mélange-maître naturel ou à base de plantes préparé ; et
b. un polymère de base ;
dans laquelle le mélange-maître à base de plantes sous la forme d'un fil de filaments ou d'une fibre discontinue et le polymère de base sont extrudés ensemble pour former la fibre non naturelle qui présente au moins l'une parmi une propriété antimicrobienne, une propriété antiodeur, une propriété de refroidissement, et,
la part en poids de l'extrait à base de plantes dans la fibre non naturelle finale est comprise entre 0,1 et 5%.

2. Composition de fibres ou de filaments non naturel(le)s selon la revendication 1, dans laquelle la fibre ou le filament non naturel(le) présente une valeur de densité linéaire comprise entre 0,33 dtex et 33,3 dtex (valeur en deniers comprise entre 0,3 DPF et 3 DPF).

3. Composition de fibres ou de filaments non naturel(le)s selon la revendication 1, dans laquelle l'extrait à base de plantes est sous forme de poudre.

4. Composition de fibres ou de filaments non naturel(le)s selon la revendication 1, dans laquelle le polymère ou le polymère de base est choisi au sein d'un groupe comportant le PET, le PBT, le PTT, le PLA, le PP, le polyamide ou un mélange de ceux-ci.

5. Composition de fibres ou de filaments non naturel(le)s selon la revendication 1, dans laquelle l'agent de couplage couple l'extrait naturel ou à base de plantes et le polymère de base.

6. Procédé de fabrication de fibres ou de filaments non naturel(le)s antimicrobien(ne)s, antiodeurs, comprenant les étapes de :
a. préparation d'un mélange-maître naturel ou à base de plantes, la préparation du mélange-maître naturel ou à base de plantes comprenant les étapes de :
i. broyer au moins un produit à base de plantes ou naturel pour produire un extrait à base de plantes ayant une taille moyenne des particules inférieure ou égale à 50 microns ; et
ii. mélanger ou mixer l'extrait à base de plantes, un agent de couplage et un polymère pour produire un mélange-maître, le rapport de l'extrait à base de plantes sur le polymère étant de 30:70 en poids dans le mélange-maître naturel ou à base de plantes préparé ; et
b. extruder ensemble le mélange-maître sous la forme d'un fil ou d'un filament ou d'une fibre et un polymère de base pour obtenir la fibre ou le filament non naturel(le) final, la quantité d'extrait à base de plantes dans la fibre ou le filament non naturel(le) final(e) est comprise entre 0,1% et 5% en poids.

7. Procédé de fabrication d'un mélange-maître naturel ou à base de plantes selon la revendication 1, comprenant les étapes de :
a. broyer au moins un produit à base de plantes ou naturel pour produire un extrait à base de plantes ayant une taille moyenne des particules inférieure ou égale à 50 microns ; et
b. mélanger ou mixer l'extrait à base de plantes, un polymère et un agent de couplage pour fabriquer un mélange-maître naturel ou à base de plantes, le rapport de l'extrait à base de plantes sur le polymère étant de 30:70 en poids dans le mélange-maître naturel ou à base de plantes préparé.

8. Procédé selon la revendication 7, dans lequel l'agent de couplage comprend au moins un agent de couplage, un stabilisant aux UV, un antioxydant, un agent antistatique ou des colorants dans un mélangeur à haute vitesse ou des mélangeurs de ceux-ci.

9. Procédé selon la revendication 7, dans lequel la fibre non naturelle fabriquée comprend au moins l'une parmi une propriété antimicrobienne, une propriété antiodeur, une propriété de refroidissement et une propriété de durabilité.

10. Procédé selon la revendication 7, dans lequel le mélange-maître à base de plantes est mélangé avec au moins un autre mélange-maître et extrudé pour introduire une fibre non naturelle.
